(19) Europäisches Patentamt
European Patent Office
Office européen des brevets

(11) **EP 3 135 326 A1**

(12) **EUROPÄISCHE PATENTANMELDUNG**

(43) Veröffentlichungstag:
01.03.2017 Patentblatt 2017/09

(51) Int Cl.:
*A61M 1/10* *(2006.01)*

(21) Anmeldenummer: **15182128.7**

(22) Anmeldetag: **24.08.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(71) Anmelder: **Berlin Heart GmbH**
**12247 Berlin (DE)**

(72) Erfinder:
• **GRAICHEN, Kurt**
**13189 Berlin (DE)**
• **WIESENER, Constantin**
**14471 Potsdam (DE)**
• **KALLENBACH, Sebastian**
**13353 Berlin (DE)**

(74) Vertreter: **Pfenning, Meinig & Partner mbB**
**Patent- und Rechtsanwälte**
**Joachimsthaler Straße 12**
**10719 Berlin (DE)**

(54) **HERZPUMPE SOWIE VERFAHREN ZUM BETRIEB EINER HERZPUMPE**

(57) Das Schutzrecht bezieht sich auf eine Herzpumpe und insbesondere ein Verfahren zum Betrieb einer VAD(ventricular assist device)-Herzpumpe (3), bei dem ein zeitabhängiger Druck des zu fördernden Blutes unmittelbar mittels wenigstens eines am Pumpeneinlass (4, 41) und/oder am Pumpenauslass (5, 42) angeordneten Drucksensors (8, 21) gemessen wird. Durch die Auswertung der gemessenen Druckverläufe und/oder entsprechender Änderungsraten des gemessenen Drucks lassen sich sowohl Förderparameter der Pumpe (3) als auch physiologische Werte des durch die Pumpe unterstützten Herzens komfortabel und zuverlässig ermitteln.

Fig. 1

EP 3 135 326 A1

**Beschreibung**

Die Erfindung liegt auf dem Gebiet der Mechanik und der Elektrotechnik und ist mit besonderem Vorteil in der Medizintechnik einsetzbar.

Insbesondere bezieht sich die Erfindung auf den Betrieb einer Herzpumpe.

Seit einigen Jahren sind Herzpumpen zur Förderung von Blut und zum Ersatz oder zur Unterstützung eines Patientenherzens bekannt. Solche Pumpen können in verschiedener Art ausgeführt und auch auf unterschiedliche Weisen betrieben werden. Sie können das Patientenherz im Wesentlichen ersetzen und dessen Funktion vollumfänglich übernehmen oder auch lediglich als Unterstützung eines nicht voll funktionsfähigen Herzens eingesetzt werden, wobei eine Restherzaktivität stattfindet und auch gefördert werden soll.

Zu dem letztgenannten Zweck sind beispielsweise sogenannte VADs (ventricular assist devices, im Folgenden: VAD-Herzpumpen) bekannt, die vorübergehend (bridge-to-recovery) oder zur Vorbereitung einer Transplantation (bridge-to-transplant) oder auch dauerhaft (destination therapy) das Herz eines Patienten unterstützen können. Dabei ist es wichtig und von großem Interesse, sowohl die Restherzfunktion des Patientenherzens als auch das Betriebsverhalten der Pumpe zu detektieren und auszuwerten.

Bisher ist es bekannt, aus Betriebsparametern der Pumpe, wie beispielsweise dem Motorstrom, der Drehzahl sowie der Lagerleistung und/oder der axialen Positionsverschiebung eines Pumpenrotors in einem belasteten Lager, Größen wie beispielsweise den durch die Pumpe erzeugten Differenzdruck und den durch die Pumpe geförderten Volumenstrom des Blutes zu bestimmen. Anhand von Referenzwerten oder Rechenmodellen kann aus dem durch die Blutpumpe geförderten Volumenstrom auf die Restherzfunktion zumindest teilweise geschlossen werden. Dadurch können physiologische Parameter des Patientenherzens zu Diagnose- und Therapiezwecken ermittelt und auch der Betrieb der Pumpe optimiert bzw. geregelt werden. Zudem können durch bestimmte Messungen Störungen an der Pumpe selbst, wie Thrombenbildung oder Strömungsanomalitäten, detektiert werden.

Die entsprechenden benötigten Betriebsparameter der Pumpe stehen nicht bei allen Pumpentypen problemlos und mit der gewünschten Genauigkeit, der gewünschten zeitlichen Auflösung, der gewünschten Empfindlichkeit oder der gewünschten Zugänglichkeit zur Verfügung. Aus diesem Grund liegt der vorliegenden Neuerung die Aufgabe zugrunde, VAD-Herzpumpen bzw. ein entsprechendes Betriebsverfahren für eine VAD-Herzpumpe derart weiterzuentwickeln, dass Parameter, die mit dem Druck des zu fördernden Blutes zusammenhängen, möglichst einfach und zuverlässig ermittelt und verarbeitet werden können.

Die Aufgabe wird mit den Merkmalen der Neuerung gemäß Patentanspruch 1 gelöst. Die Unteransprüche 2 bis 14 geben vorteilhafte Ausgestaltungen der Herzpumpe an.

Die Neuerung bezieht sich außerdem auf ein Verfahren nach Patentanspruch 15.

Dementsprechend bezieht sich die Neuerung auf eine Blutpumpe bzw. ein Verfahren zum Betrieb einer VAD(ventricular assist device)-Herzpumpe, bei dem ein zeitabhängiger Druck des zu fördernden Blutes unmittelbar mittels wenigstens eines am Pumpeneinlass und/oder am Pumpenauslass angeordneten Drucksensors gemessen wird.

Es sei ausdrücklich betont, dass das vorliegende Schutzrecht sich sowohl auf eine Blutpumpe selbst als auch auf eine Regeleinrichtung zum Betrieb einer solchen Pumpe, ein Betriebsverfahren und/oder Regelverfahren für die Pumpe sowie ein Computerprogrammprodukt bezieht, welches eingerichtet ist, eine Blutpumpe bzw. Regeleinrichtung entsprechend anzuweisen und zu steuern.

Die im Folgenden genannten Verfahrensschritte beziehen sich auf sämtliche der obigen Gegenstände.

Durch die unmittelbare Messung des zeitabhängigen Drucks des zu fördernden Blutes mittels eines Sensors können die Druckwerte mit der Genauigkeit, Häufigkeit und Reproduzierbarkeit gemessen werden, wie sie für den jeweiligen Verwendungszweck erforderlich sind. Entsprechende Sensoren sind kommerziell verfügbar. Sie können die Druckwerte mittels elektrischer Signale ausgeben. Ein derartiger Drucksensor kann auch zusätzlich einen Mikrocontroller zur Vorverarbeitung der Druckwerte aufweisen.

Die Anordnung am Pumpeneinlass oder -auslass kann derart vorgesehen sein, dass der Sensor unmittelbar an der Pumpe befestigt ist. Der Sensor kann in einem Pumpeneinlass- oder -auslassstutzen oder an einem solchen Stutzen vorgesehen sein. Derartige Anschlussstutzen sind starr und unelastisch ausgebildet, so dass der Betrieb des Sensors auch in einer gewissen Entfernung von der Pumpe den Druck unmittelbar an der Pumpe widerspiegelt. In diesem Sinne ist die Wortwahl "am Pumpeneinlass und/oder Pumpenauslass" zu verstehen.

Eine entsprechend zuverlässige und unmittelbare Erfassung der Druckwerte erlaubt die Verwendung für verschiedenartige Steuer- und Regelverfahren zum Betrieb der Pumpe und auch zur Ermittlung physiologischer Werte des Patienten, die mittelbar oder unmittelbar in die Steuerung und/oder Regelung der Pumpe einbezogen werden. In oder an den Drucksensoren können zusätzlich auch Temperatursensoren angeordnet sein. Durch eine Berücksichtigung der Temperatur kann eine Kompensation des Temperatureinflusses auf die Druckmessung vorgenommen werden.

Eine vorteilhafte Ausgestaltung des Verfahrens kann vorsehen, dass laufend einer oder mehrere der folgenden Parameter bestimmt wird:

-    die Änderungsrate des gemessenen Drucks pro Zeiteinheit,

- das Maximum und/oder Minimum der Änderungsrate des gemessenen Drucks innerhalb einer Zeitperiode, insbesondere innerhalb einer oder mehrerer Herzperioden,

- das Maximum und/oder Minimum des Zeitverlaufs des gemessenen Drucks innerhalb einer Zeitperiode, insbesondere innerhalb einer oder mehrerer Herzperioden,

- die Herzfrequenz und/oder Arrhythmien durch den Verlauf der erfassten Druckwerte.

Eine weitere vorteilhafte Ausgestaltung sieht vor, dass laufend die Änderungsrate des gemessenen Drucks pro Zeiteinheit bestimmt und das Maximum und/oder das Minimum dieser Änderungsgröße innerhalb einer Zeitperiode ermittelt wird. Insbesondere wird die Änderungsrate innerhalb einer Herzperiode ermittelt. Unter der Änderungsrate des gemessenen Drucks ist die erste zeitliche Ableitung der Druckwerte zu verstehen. Das Maximum der ersten Ableitung des Drucks nach der Zeit im Ventrikel dient zur Ermittlung der Kontraktilität des Herzens. Da aus dem am Pumpeneinlass unmittelbar gemessenen Druck auf den im Ventrikel herrschenden Druck geschlossen werden kann, kann mittels der durch den Drucksensor gemessenen Werte das Maximum sowie das Minimum der Zeitableitung des Drucks im Ventrikel geschlossen werden.

Es kann zudem vorgesehen sein, dass aus den gemessenen Druckwerten über mehr als eine Periode des Herzrhythmus die Vorlast und die Nachlast des Herzens bestimmt wird.

Es kann dann im nächsten Schritt vorgesehen sein, dass aus den gemessenen und bestimmten Größen die Kontraktilität des Herzens ermittelt wird.

Zur Durchführung der entsprechenden Auswertungen in Bezug auf die Kontraktilität vgl, den Artikel von Sarazan, Kroehle und Main, Left ventricular pressure, contractility and dP/dt(max) in nonclinical drug safety assessment studies, Journal of Pharmacological and Toxicological Methods, 2012.

Die Kontraktilität ist ein wichtiger Parameter der Restherzaktivität, der Aufschluss über eventuelle Änderungen der Leistungsfähigkeit des Patientenherzens geben kann. Somit lassen sich auch Rückschlüsse auf eine mögliche Entwöhnung des Patienten von der Herzpumpenunterstützung gewinnen (Weaning). Die Verbesserung bzw. die Erholung der Kontraktilität ist Voraussetzung für das Weaning. Ein ermittelter Indikator der Kontraktilität kann daher ausgegeben und/oder für die Einleitung eines Weaning-Prozesses oder für die Überwachung des Erfolges eines Weaning-Prozesses verwendet werden.

Es kann auch vorgesehen sein, dass durch den Verlauf der erfassten Druckwerte die Herzfrequenz und/oder Arrhythmien ermittelt werden. Zudem kann aus den gemessenen und bestimmten Größen die Amplitude und/oder Pulsatilität des Ventrikeldrucks bestimmt werden.

Eine weitere vorteilhafte Ausgestaltung sieht vor, dass der am Pumpeneinlass zwischen der Pumpe und dem Ventrikel gemessene Druck laufend mit einem Schwellenwert verglichen wird, der kleiner ist als die im Normalbetrieb auftretenden Druckwerte, und dass bei Unterschreiten der Schwelle ein Ansaugvorgang erkannt und insbesondere unmittelbar die Pumpleistung verringert wird. Dabei kann der Schwellenwert von einer aktuell erfassten Drehzahl der Pumpe abhängen.

Eine mögliche Störung bei dem Betrieb einer Herzunterstützungspumpe in Form einer VAD-Pumpe besteht darin, dass sich der Ansaugstutzen der Pumpe, der in eine Herzkammer hineinragt, an einer Herzwand festsaugen kann. Nähert sich die ansaugende Einlasskanüle der Pumpe durch eine Bewegung des Patienten oder durch sonstige Umstände (beispielsweise geringe Ventrikelfüllung) bis zu einem gewissen Abstand an eine Wand des Herzens an, so bewirkt der Ansaugunterdruck ein weiteres Ansaugen an dem Gewebe der Herzwand.

In der Vergangenheit wurden solche Ansaugzustände durch Beobachtung des Differenzdrucks über die Pumpe und der Betriebsparameter der Pumpe detektiert. Eine solche Detektion ist jedoch nicht immer einfach, da auch andere Vorgänge als ein Ansaugen entsprechende kurzfristige Druckentwicklungen erzeugen können. Außerdem stellen die für die Ansaugdetektion verwendeten Parameter keine direkten, sondern indirekte Messgrößen dar. Mithilfe einer unmittelbaren Druckmessung mit einem Drucksensor kann jedoch ein Unterdruck mit weitaus größerer Sicherheit entdeckt und identifiziert werden. Wird als Schwelle ein Druck unterhalb einer bestimmten, im Normalbetrieb nicht unterschrittenen Druckgrenze erreicht, so ist mit großer Sicherheit zu entscheiden, dass bei Unterlaufen der Schwelle ein Ansaugvorgang vorliegt oder in Kürze bevorsteht, da ein derart geringer Druck nicht auf andere Weise als durch ein Ansaugen auftreten kann. Dadurch kann ein Ansaugen bereits vor dem vollständigen Verschluss der Einlasskanüle erkannt und daher präventiv verhindert werden. Zur Beseitigung des unerwünschten Zustands kann die Pumpleistung kurzfristig verringert werden, so dass das Ansaugende sich von der Herzwand lösen kann.

Eine weitere vorteilhafte Ausgestaltung kann vorsehen, dass mittels zweier Drucksensoren der Druck des zu fördernden Blutes am Einlass und am Auslass der VAD-Pumpe erfasst wird, dass ein Zielwert für den absoluten Druckunterschied zwischen dem Einlass und dem Auslass der Pumpe vorgegeben wird und dass die Pumpleistung derart geregelt wird, dass der Zielwert erreicht wird. Durch die Regelung einer derartigen Druckdifferenz über der Pumpe kann künstlich ein bestimmter virtueller Strömungswiderstand über der Pumpe eingestellt werden, der bis zu einer Vollsperrung der Pumpe reichen und damit eine Klappenschüeßung innerhalb des Pumpenpfades simulieren kann. Das durch die Restherzaktivität des Herzens geförderte Blut wird dann ausschließlich durch die Aorta ausgeworfen. Hierdurch können für die Restherzaktivität bestimmte Bedingungen geschaffen werden, die die Restherzaktivität fördern. Die Regelung eines bestimmten Differenzdrucks kann auch zur Durchführung physiologischer Messungen am Herzen dienen.

Die Neuerung kann vorteilhaft auch derart ausgestaltet werden, dass mittels zweier Drucksensoren der Druck des zu fördernden Blutes am Einlass und am Auslass der VAD-Pumpe erfasst und hieraus die tatsächliche Druckdifferenz über der Pumpe bestimmt wird, dass anhand von erfassten Betriebsparametern der Pumpe durch Vergleich mit Referenzwerten ein theoretischer Wert der Druckdifferenz ermittelt und mit dem tatsächlichen Wert der Druckdifferenz verglichen wird und dass bei Abweichungen das Vorliegen einer Störung der Pumpe signalisiert wird.

Durch den Vergleich der Druckdifferenz, die theoretisch gemäß den Betriebsparametern der Pumpe, der Pumpleistung bzw. dem Drehmoment des Pumpenrotors, der Drehzahl, der Rotorposition innerhalb des Magnetlagers und anderen einschlägigen Größen erreicht werden müsste, mit der tatsächlich erzielten und direkt gemessenen Druckdifferenz lässt sich die Leistungsfähigkeit der Pumpe im Vergleich zu einem Referenzwert, beispielsweise bei Inbetriebnahme der Pumpe, feststellen. Liegt die Leistungsfähigkeit der Pumpe unterhalb eines Referenz- oder Anfangswerts, so lässt dies auf eine Verschlechterung der Pumpengeometrie, Verformung, Thrombenbildung oder dergleichen schließen, oder auf die Ausbildung einer stationären Strömung in Form eines Wirbels oder einer ähnlichen Erscheinung, die den Durchfluss des Blutes durch die Pumpe hemmt. Zudem kann auch ein Lagerschaden zu einer solchen Leistungsminderung der Pumpe führen. Derartige Zustände lassen sich durch den genannten Vergleich unmittelbar nachweisen und signalisieren, so dass die Pumpe gewartet oder ausgetauscht werden kann.

Eine weitere vorteilhafte Ausgestaltung der Erfindung kann vorsehen, dass durch den Verlauf der erfassten Druckwerte die Herzfrequenz ermittelt wird. Des Weiteren können hieraus Arrhythmien der Herzfrequenz ermittelt werden.

Ein vorteilhaftes Verfahren kann zudem vorsehen, dass aus der erfassten Herzfrequenz HR der Zeitdifferenz ED zwischen dem Zeitpunkt $t_1$ (dP/dt$_{max}$), zu dem die Druckänderungsgeschwindigkeit ihr Maximum erreicht, und dem Zeitpunkt $t_2$ (dP/dt$_{min}$), zu dem die Druckänderungsgeschwindigkeit ihr Minimum erreicht, dem Druck $P_{1st}$ zum Zeitpunkt des maximalen Blutstroms und dem Druck $P_{ES}$ zum Zeitpunkt $t_2$ das Herzzeitvolumen (cardiac output) HZV gemäß der Formel

$$HZV = HR \frac{\left(P_{1st} - P_{ES}\right) ED}{2 Z_C}$$ bestimmt wird. Der beschriebenen Bestimmung des Herzzeitvolumens liegt eine lineare Näherung zugrunde, die davon ausgeht, dass der Volumenstrom durch die Pumpe in erster Näherung proportional zum Ventrikeldruck ist. Dieses Modell lässt Elastizitätseffekte des Blutkreislaufs außer Acht.

Es kann zudem vorgesehen sein, dass aus dem Ventrikeldruckverlauf, dem Differenzdruck über der Pumpe und/oder dem zeitabhängigen Druckabfall über der Auslasskanüle der Druckverlauf in der Aorta und aus diesem die Auswurffraktion bestimmt wird (ejection fraction). Da die Auswurffraktion aus dem Herzzeitvolumen unter Berücksichtigung des enddiastolischen Restvolumens des Herzens bestimmbar ist, kann somit die Auswurffraktion unter Verwendung der kontinuierlich erfassten Druckwerte im Ventrikel / am Pumpeneinlass ermittelt werden.

Gegenstand der vorliegenden Anmeldung ist zudem eine Herzpumpeneinrichtung mit einer VAD-Herzpumpe, einem Steuergerät und wenigstens einem Drucksensor am Pumpeneinlass und/oder am Pumpenauslass zur Durchführung des Verfahrens nach einem der Ansprüche 1 bis 13.

Im Folgenden wird anhand von Ausführungsbeispielen in Figuren einer Zeichnung die Neuerung gezeigt und erläutert. Dabei zeigt

Fig. 1 schematisch die Anordnung einer Herzunterstützungspumpe im Körper eines Patienten,

Fig. 2 einen erfassten Druckverlauf im Bereich der Pumpe,

Fig. 3 das Ansaug-Ende einer mit einer Herzunterstützungspumpe verbundenen Kanüle in einer Herzkammer,

Fig. 4 eine Herzunterstützungspumpe mit zwei Drucksensoren,

Fig. 5 die grundsätzlichen Funktionselemente einer typischen Herzunterstützungspumpe,

Fig. 6 drei Diagramme zu einem Modell für die Bestimmung des Herzzeitvolumens aus Ventrikeldruckmessungen sowie

Fig. 7 ein Diagramm, das die Entwicklung des Ventrikeldrucks über zwei Diastolen und eine dazwischenliegende Systole darstellt.

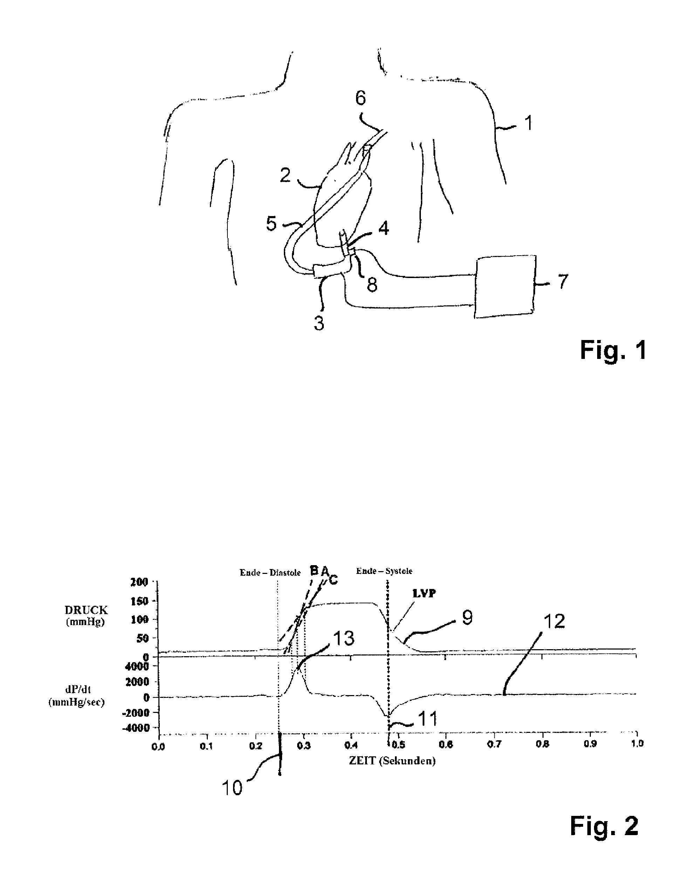

Figur 1 zeigt schematisch den Oberkörper 1 eines Patienten mit einer schematischen Darstellung des Herzens 2 sowie einer Herzpumpe 3. Die Herzpumpe 3 ist über eine Kanüle 4 auf der Ansaugseite mit einem Ventrikel des Herzens 2 verbunden und saugt dort das zu fördernde Blut über einen Pumpeneinlass an. Das Blut wird durch die Pumpe 3 gefördert, durch die Auslasskanüle 5 zur Aorta 6 gefördert und in die Aorta eingeleitet.

Das Herz 2 des Patienten kann neben der Pumpfunktion der Herzpumpe 3 selbst im Rahmen der sogenannten Restherzfunktion Blut durch Kontraktion des Herzmuskels in die Aorta fördern, so dass das Herz 2 und die Herzpumpe 3 parallel und einander unterstützend arbeiten.

Die Herzpumpe 3 ist mit einer Steuereinrichtung 7 verbunden, die die Herzpumpe mit Energie versorgt und entsprechend einem Steuer- oder Regelverfahren ansteuert. Die Steuereinrichtung 7 ist zudem mit einem Drucksensor 8 verbunden, der im Bereich der Ansaugkanüle 4 am Pumpeneinlass 41 im Blutstrom angeordnet ist und den Druck des zu fördernden Blutes in der Ansaugkanüle signalisiert. Der Drucksensor 8 meldet Druckwerte mittels elektrischer Signale an die Steuereinrichtung 7. Durch die Rückmeldung über den tatsächlichen Druckwert zwischen dem Ventrikel und der Pumpe 3 werden verschiedene vorteilhafte Merkmale erfindungsgemäßer Ausgestaltungen realisierbar.

Figur 2 zeigt in einem Diagramm auf der horizontalen x-Achse einen Zeitmaßstab in Sekunden und im oberen Teil eine Kurve, in der der Druck des Blutes, genauer die Druckdifferenz zum Atmosphärendruck, gegenüber der Zeit aufgetragen ist. Die Druckkurve ist mit 9 bezeichnet.

In einer ersten Phase bis zum Zeitpunkt 10 bleibt der Druck auf einem geringen und nahezu konstanten Niveau, wobei der Zeitpunkt 10 das Ende der Diastole repräsentiert. Danach steigt der Druck durch Kontraktion des Herzens an, um zum Ende der Systole, d. h. zum Zeitpunkt 11, wieder abzufallen. In der Figur sind an der Kurve 9 im Bereich des Druckanstiegs drei Tangenten A, B und C an die Kurve gelegt, die zu verschiedenen Zeitpunkten verschiedene Steigungen des Druckverlaufs zeigen.

Gemäß dem erfindungsgemäßen Verfahren kann der Flüssigkeitsdruck mit hoher zeitlicher Auflösung bestimmt werden, so dass auch die zeitlichen Änderungen des Drucks, d. h. die Steigungen der Druckkurve, mit entsprechender Auflösung ermittelbar sind.

In dem Diagramm der Figur 2 sind im unteren Teil unter der Druckkurve 9 Werte für die zeitliche Ableitung des Drucks in einer Kurve 12 dargestellt. Es zeigt sich, dass nach dem Zeitpunkt 10 zunächst nicht nur der Druck ansteigt, sondern auch die Raten des Druckanstiegs bis zu einem Maximum 13 ansteigen. Dieser Wert entspricht $dP/dt_{max}$, d. h. dem Maximum der ersten Ableitung des Drucks. Dieses Maximum liegt nach dem Ende der Diastole. In dem Zeitbereich zwischen dem Zeitpunkt 10 und dem Zeitpunkt 11 tritt nach Erreichen des Maximums eine Phase mit geringen Änderungsraten des Drucks ein, wobei zum Ende der Systole bei Annäherung an den zweiten Zeitpunkt 11 der Wert für dP/dt ein Minimum $dP/dt_{min}$ erreicht.

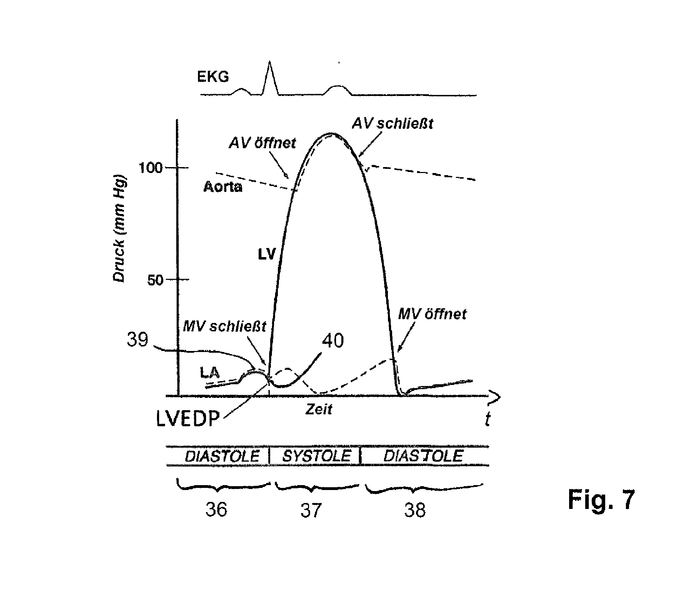

Der Wert $dP/dt_{max}$ dient nach Sarazan, Kroehle und Main, 2012 der Abschätzung der Kontraktilität bei der Restherzfunktion. Um nach dem genannten Verfahren die Kontraktilität ermitteln zu können, ist zumindest auch die Vorlast des Ventrikel aufzuzeichnen. Als Vorlast kann in einer vereinfachten Vorstellung der enddiastolische Druck im Herzen verstanden werden. In diesem Zusammenhang wird auf die Erläuterungen zu Figur 7 verwiesen.

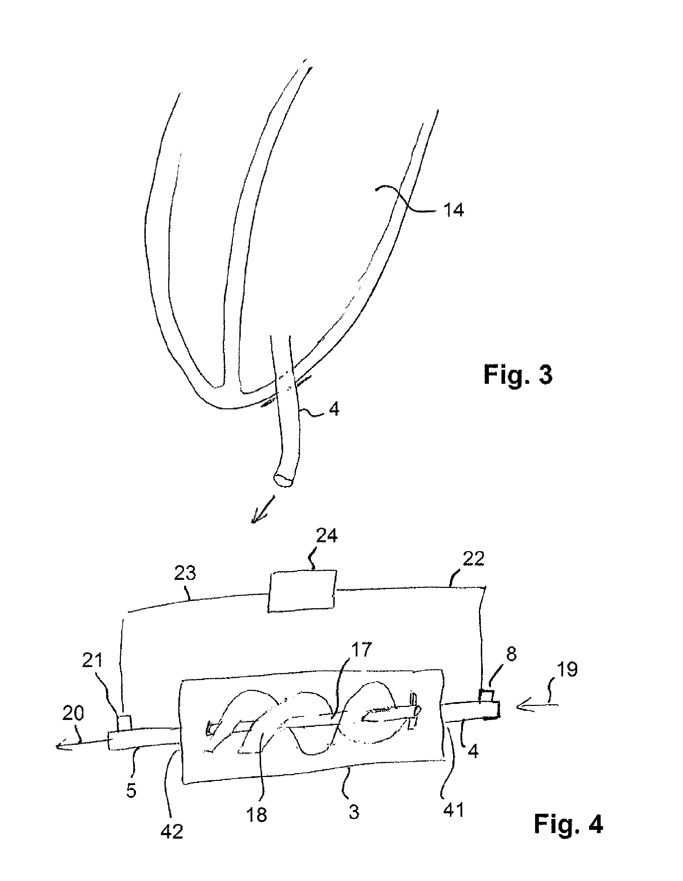

Figur 3 dient der Darstellung des Phänomens des Ansaugens in der Herzkammer. Es ist eine Ansaugkanüle 4 in schematischer Form dargestellt. Durch Ansaugen an die Herzwand kann die Ansaugöffnung wenigstens teilweise verschlossen und dadurch verstopft werden.

Durch die unmittelbare Druckmessung im Bereich der Ansaugkanüle 4 bzw. der Einlassöffnung der Pumpe 3 kann die Druckentwicklung zeitlich hochauflösend verfolgt werden, so dass auf den Druckabfall schnell reagiert und die Leistung der Pumpe verringert werden kann. Darauf kann sich die Ansaugöffnung der Kanüle 4 von der Herzwand lösen. Die unmittelbare Erfassung von Druckwerten mittels eines Drucksensors erlaubt nicht nur zeitlich hochaufgelöste Messungen, sondern auch unmissverständliche Interpretationen der Druckwerte und eine zuverlässige Reaktion bei Unterlaufen entsprechend gesetzter Schwellenwerte. Dadurch kann ein Ansaugen bereits vor dem vollständigen Verschluss der Einlasskanüle erkannt und ein Ansaugen daher präventiv verhindert werden.

Figur 4 zeigt schematisch einen Längsschnitt durch eine Herzpumpe 3 mit einer Nabe 17 sowie Förderelementen in Form von schraubenförmigen Schaufeln 18. Es ist eine Einlasskanüle/Ansaugkanüle 4 dargestellt, durch die in Richtung des Pfeils 19 Blut angesaugt wird, sowie eine Auslasskanüle 5, die am Pumpenauslass 42 angeschlossen ist und durch die in Richtung des Pfeils 20 das Blut ausgestoßen wird. Bei dem gezeigten Ausführungsbeispiel ist in der Ansaugkanüle 4 ein erster Drucksensor 8 angeordnet, während in der Auslasskanüle 5 ein zweiter Drucksensor 21 angeordnet ist. Die beiden Drucksensoren können auch unmittelbar am jeweiligen Ende der Nabe 17 angeordnet sein. Beide Drucksensoren 8, 21 sind über Signalleitungen 22, 23 mit einer Steuereinrichtung 24 zur Steuerung der Pumpe 3 verbunden.

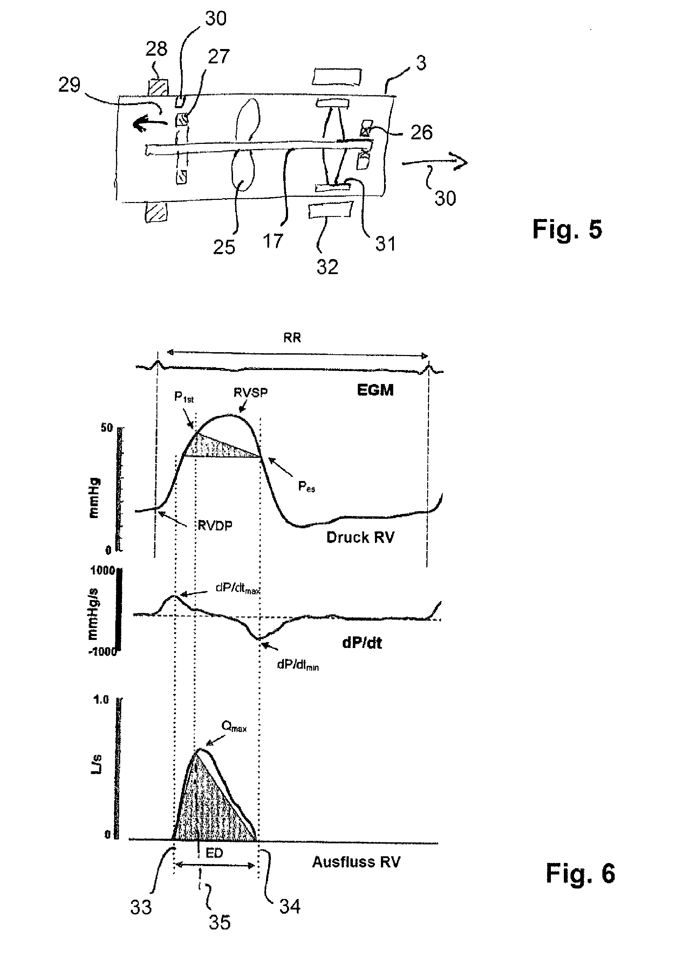

In Figur 5 sind der mechanische Aufbau, die Lagerung und der Antrieb der Pumpe 3 schematisch dargestellt. Grundsätzlich sind im Rahmen der Neuerung vielfältige Pumpenkonstruktionen einsetzbar, wie zum Beispiel reine Axial-Rotorpumpen, aber auch Axial/Radial-Rotorpumpen. Die Nabe des Rotors ist in Figur 5 mit 17 bezeichnet, wobei nur stellvertretend ein Förderelement 25 des Rotors in Form einer Förderschaufel dargestellt ist. Die Förderelemente sind jeweils mit der Nabe 17 des Rotors fest verbunden. Die Nabe 17 ist in einem ersten Radiallager 26 in radialer Richtung gelagert. Das Radiallager 26 kann beispielsweise als hydrodynamisches Lager ausgebildet sein. Zudem ist an dem gegenüberliegenden Ende der Nabe 17 diese mit einem Magnetring 27 verbunden, der zur Bildung eines Axial-/Radiallagers mit einem ortsfesten Magnetring 28 zusammenwirkt. Eine axiale Abstoßung zwischen den Magnetringen 27, 28 bewirkt das Auffangen von Axialkräften, die dadurch, dass der Rotor das Blut in Richtung des Pfeils 30 fördert, den Rotor in Richtung des Pfeils 29 drücken. Die aufzufangende Axialkraft auf den Rotor entspricht insoweit der Reaktionskraft des zu fördernden Blutes. Die Abstoßungskraft zwischen den magnetischen Ringen 27 und 28 bewirkt nicht nur eine Lagerung in axialer Richtung, sondern gegebenenfalls, soweit nicht ein zusätzliches Radiallager vorgesehen ist, auch in radialer Richtung. Die magnetische Abstoßungskraft des ortsfesten Magnetrings 28 ist wenigstens teilweise mittels eines durch einen Elektromagneten erzeugten Magnetfelds bewirkt. Durch entsprechende Ansteuerung der Spule des Elektromagneten kann eine Regelung des Magnetlagers bewirkt werden, um die Axialposition des Rotors bzw. des Magnetrings 27 möglichst konstant zu halten. Zur Bildung eines geschlossenen Regelkreises wird die Axialposition des Magnetrings 27 mittels eines Positionssensors 30 detektiert.

Die Stromstärke, mit der das Magnetlager 28 angesteuert werden muss, um die Axialkraft zum Halten des Rotors in Axialrichtung aufzubringen, kann zur Berechnung des durch die Pumpe erzeugten Differenzdrucks verwendet werden. Hierzu können auch das Drehmoment, das auf den Rotor wirkt, sowie die Drehzahl des Rotors herangezogen werden.

Der Rotor wird mittels eines elektromotorischen Antriebs rotierend angetrieben, wobei ein Rotorteil 31 Permanentmagnete umfasst, die im Feld eines Stators 32 in Umfangsrichtung angetrieben werden. Der Stator 32 weist Wicklungen auf, die im Rahmen der Ansteuerung eines bürstenlosen Motors beispielsweise durch pulsweitenmodulierte Signale angesteuert werden können. Die Überwachung der Statorströme erlaubt eine Drehmomentbestimmung.

Anhand der Figur 6 wird ein Modell zur Berechnung/Abschätzung des Herzzeitvolumens beschrieben. Das Herzzeitvolumen gibt an, wie viel Blut über die Aorta in den Körperkreislauf gefördert wird. Über die Bestimmung der Pumpenparameter ist lediglich der durch die Pumpe geförderte Fluss ermittelbar. Durch die Betrachtung der Druck- und Druckänderungsgrößen im Bereich des Herzens kann der Unterstützungsgrad bzw. das Herzzeitvolumen des Herzens selbst ermittelt werden. Dies geschieht anhand der sogenannten Pulskonturanalyse. Es wird dabei der kontinuierliche Verlauf des arteriellen Drucks und daraus das Herzzeitvolumen bestimmt. Dabei wird ein lineares Modell, d. h. ein linearer Zusammenhang zwischen dem Ventrikeldruck und der geförderten Blutmenge, zugrunde gelegt. Das geförderte Herzzeitvolumen ergibt sich in einer Abschätzung aus der folgenden Formel:

$$HZV = HR \frac{(P_{1st} - P_{ES})\,ED}{2\,Z_C}$$

Dabei ist HZV das geschätzte Herzzeitvolumen, HR die Herzrate, $Z_C$ die patientenspezifische Impedanz der Aorta, und die Größen $P_{1st}$, $P_{ES}$ und ED ergeben sich aus Figur 6. Dabei werden die wissenschaftlichen Erkenntnisse von Valsecchi et al., Cardiac output derived from left ventricular pressure during conductance catheter evaluations: an extended model flow method, Journal of Clinical Monitoring and Computing, 2007 und Karamanoglu, Estimation of cardiac output in patients with congestive heart failure by analysis of right ventricular pressure waveforms, Biomedical Engineering Online, 2011 zugrunde gelegt. Im oberen Teil der Figur 6 ist hierzu der Druckverlauf des Ventrikeldrucks etwa vom Ende einer Diastole bis zum Ende der nächsten Diastole dargestellt, Der Ventrikeldruck erhöht sich dabei bis zu einem Maximum und fällt dann zum Ende der Systole ab.

Derselbe Zeitraum ist in der mittleren Darstellung der Figur 6 anhand eines Diagramms dargestellt, in dem die Druckänderungen des Ventrikeldrucks als zeitliche Ableitung des erfassten Druckwerts dargestellt sind. Dabei ergibt sich der Zeitpunkt 33 als der Zeitpunkt, zu dem das Maximum $dP/dt_{max}$ in der mittleren Darstellung der Figur 6 erreicht wird. Mit 34 ist der Zeitpunkt bezeichnet, zu dem in dem mittleren Diagramm der Figur 6 das Minimum $dP/dt_{min}$ der Druckänderung erreicht wird. Als Differenz zwischen den Zeitpunkten 33 und 34 ergibt sich die Zeitdauer EB. Mit $P_{ES}$ ist der Druckwert bezeichnet, der zum Zeitpunkt 34 erreicht ist.

Im unteren Diagramm der Figur 6 ist der Fluss durch die Aorta in Litern pro Sekunde über der Zeit zwischen den Zeitpunkten 33 und 34 aufgetragen. In die den Fluss bezeichnende Kurve wird ein maximal großes Dreieck einbeschrieben. Die obere Spitze des Dreiecks definiert den Zeitpunkt 35, zu dem der Druckwert $P_{1st}$ erfasst wird. Damit sind alle Größen definiert, die in die oben genannte Gleichung zur Ermittlung des Herzzeitvolumens einzusetzen sind.

Anhand der Figur 7 sei noch das Verfahren zur Ermittlung der Vor- und Nachlast anhand einer kontinuierlichen Absolutdruckmessung beschrieben. Das Diagramm zeigt den Druckverlauf, wobei der Druck auf der y-Achse und die Zeit auf der horizontalen x-Achse aufgetragen ist. Es werden bezüglich der Zeit eine erste Diastole 36, eine darauf folgende Systole 37 und eine hierauf folgende weitere Diastole 38 betrachtet. Zunächst durchläuft zum Ende der ersten Diastole 36 der Druck ein relatives Maximum 39, bevor ein scharfes, V-förmiges Minimum durchlaufen wird und der Druck darauf in der Systole stark ansteigt. Der Druckwert des Minimums 40 stellt den linksventrikulären, enddiastolischen Druck dar und ist damit ein Maß für die Vorlast. Dieser Wert kann, wie oben im Zusammenhang mit Figur 2 beschrieben, unter anderem zur Ermittlung des Herzzeitvolumens verwendet werden.

Es sollen in diesem Zusammenhang noch drei Aspekte der Neuerung hervorgehoben werden, die jeweils allein für sich ohne den Rückbezug auf den Patentanspruch 1 eine Erfindung darstellen können: ein Verfahren zum Betrieb einer VAD-Herzpumpe, bei dem mittels zweier Drucksensoren der Druck des zu fördernden Blutes am Einlass und am Auslass der VAD-Pumpe erfasst und hieraus die tatsächliche Druckdifferenz bestimmt wird, bei dem anhand von erfassten Betriebsparametern der Pumpe durch Vergleich mit Referenzwerten ein theoretischer Wert der Druckdifferenz ermittelt und mit dem tatsächlichen Wert der Druckdifferenz verglichen wird und bei dem bei Abweichungen das Vorliegen einer Störung der Pumpe signalisiert wird; ein Verfahren zum Betrieb einer VAD-Herzpumpe, bei dem absolute Druckwerte des zeitabhängigen Drucks des zu fördernden Blutes durch einen Drucksensor am Einlass der Pumpe oder zwischen dem Einlass der Pumpe und dem Ventrikel erfasst werden und durch den Verlauf der erfassten Druckwerte die Herzrate ermittelt wird; sowie ein Verfahren zum Betrieb einer VAD-Herzpumpe, bei dem aus der erfassten Herzfrequenz HR, der Zeitdifferenz ED zwischen dem Zeitpunkt $t_1$ (dP/dt$_{max}$), zu dem die Druckänderungsgeschwindigkeit ihr Maximum erreicht, und dem Zeitpunkt $t_2$ (dP/dt$_{min}$), zu dem die Druckänderungsgeschwindigkeit ihr Minimum erreicht, dem Druck $P_{1st}$ zum Zeitpunkt des maximalen Blutstroms und dem Druck $P_{ES}$ zum Zeitpunkt $t_2$ das Herzzeitvolumen (cardiac output) HZV gemäß der Formel

$$HZV = HR \frac{(P_{1st} - P_{ES})ED}{2Z_C}$$

bestimmt wird.

Hierbei kann die unabhängige Erfindung weiter dadurch ausgestaltet werden, dass aus dem Ventrikeldruckverlauf der Druckverlauf in der Aorta und aus diesem das enddiastolische Volumen des Herzens bestimmt und insbesondere unter Verwendung dieses Wertes die Auswurffraktion bestimmt wird.

**Patentansprüche**

1. Blutpumpe, insbesondere VAD (ventricular assist device)-Herzpumpe (3), welche eingerichtet ist, einen zeitabhängigen Druck des zu fördernden Blutes unmittelbar mittels wenigstens eines am Pumpeneinlass (4, 41) und/oder am Pumpenauslass (5, 42) angeordneten Drucksensors (8, 21) zu messen.

2. Blutpumpe nach Anspruch 1, derart eingerichtet, dass laufend einer oder mehrere der folgenden Parameter bestimmt werden:

   - die Änderungsrate des gemessenen Drucks pro Zeiteinheit,
   - das Maximum und/oder Minimum der Änderungsrate des gemessenen Drucks innerhalb einer Zeitperiode, insbesondere innerhalb einer oder mehrerer Herzperioden,
   - das Maximum und/oder Minimum des Zeitverlaufs des gemessenen Drucks innerhalb einer Zeitperiode, insbesondere innerhalb einer oder mehrerer Herzperioden,
   - die Herzfrequenz und/oder Arrhythmien durch den Verlauf der erfassten Druckwerte.

3. Blutpumpe nach Anspruch 1, derart eingerichtet, dass laufend die Änderungsrate des gemessenen Drucks pro Zeiteinheit bestimmt und laufend das Maximum und/oder Minimum dieser Änderungsgröße innerhalb einer Zeitperiode, insbesondere innerhalb einer Herzperiode, ermittelt wird.

4. Blutpumpe nach Anspruch 2 oder 3, derart eingerichtet, dass zudem aus den gemessenen Druckwerten über mehr als eine Periode des Herzrhythmus die Vorlast und/oder die Nachlast des Herzens bestimmt wird.

5. Blutpumpe nach Anspruch 2, 3 oder 4, derart eingerichtet, dass aus den gemessenen und bestimmten Größen die Kontraktilität des Herzens ermittelt wird.

6. Blutpumpe nach Anspruch 2, 3 oder 4, derart eingerichtet, dass der Indikator der Kontraktilität für die Einleitung eines Weaning-Prozesses und/oder die Überwachung des Erfolges eines Weaning-Prozesses verwendet wird.

7. Blutpumpe nach Anspruch 1 oder einem der folgenden, derart eingerichtet, dass durch den Verlauf der erfassten Druckwerte die Herzfrequenz und/oder Arrhythmien ermittelt werden.

8. Blutpumpe nach Anspruch 2 oder einem der folgenden, derart eingerichtet, dass aus den gemessenen und bestimmten Größen die Amplitude und/oder Pulsatilität des Ventrikeldrucks ermittelt wird.

9. Blutpumpe nach Anspruch 1 oder einem der folgenden, derart eingerichtet, dass der am Pumpeneinlass (4, 41) zwischen der Pumpe (3) und dem Ventrikel gemessene Druck laufend mit einem Schwellenwert verglichen wird, der kleiner ist als die im Normalbetrieb auftretenden Druckwerte, und dass bei Unterschreiten der Schwelle ein Ansaugvorgang erkannt und insbesondere unmittelbar die Pumpleistung verringert wird, wobei der Schwellenwert insbesondere von der Drehzahl der Pumpe abhängt.

10. Blutpumpe nach Anspruch 1 oder einem der folgenden, derart eingerichtet, dass mittels zweier Drucksensoren (8, 21) der Druck des zu fördernden Blutes am Einlass und am Auslass der VAD-Pumpe (3) erfasst wird, dass ein Zielwert für den absoluten Druckunterschied zwischen dem Einlass und dem Auslass der Pumpe (3) vorgegeben wird und dass die Pumpleistung derart geregelt wird, dass der Zielwert erreicht wird.

11. Blutpumpe nach Anspruch 1 oder einem der folgenden, derart eingerichtet, dass mittels zweier Drucksensoren (8, 21) der Druck des zu fördernden Blutes am Einlass und am Auslass der VAD-Pumpe (3) erfasst und hieraus die tatsächliche Druckdifferenz bestimmt wird, dass anhand von erfassten Betriebsparametern der Pumpe (3) durch Vergleich mit Referenzwerten ein theoretischer Wert der Druckdifferenz ermittelt und mit dem tatsächlichen Wert der Druckdifferenz verglichen wird und dass bei Abweichungen das Vorliegen einer Störung der Pumpe signalisiert wird.

12. Blutpumpe nach Anspruch 1 oder einem der folgenden, derart eingerichtet, dass aus der erfassten Herzfrequenz HR, der Zeitdifferenz ED zwischen dem Zeitpunkt $t_1$ (dP/dt$_{max}$), zu dem die Druckänderungsgeschwindigkeit ihr Maximum erreicht, und dem Zeitpunkt $t_2$ (dP/dt$_{min}$), zu dem die Druckänderungsgeschwindigkeit ihr Minimum erreicht, dem Druck $P_{1st}$ zum Zeitpunkt des maximalen Blutstroms und dem Druck $P_{ES}$ zum Zeitpunkt $t_2$ der Cardiac Output (das Herzzeitvolumen, HZV) gemäß der Formel

$$HZV = HR \frac{\left(\dot{P}_{1st} - \dot{P}_{ES}\right)ED}{2Z_C}$$

bestimmt wird.

13. Blutpumpe nach Anspruch 10, derart eingerichtet, dass aus dem Ventrikeldruckverlauf, dem Differenzdruck über der Pumpe und dem zeitabhängigen Druckabfall über der Auslasskanüle der Druckverlauf in der Aorta und aus diesem die Auswurffraktion bestimmt wird.

14. Blutpumpe nach einem der vorhergehenden Ansprüche, enthaltend ein Steuergerät (7, 24) und wenigstens einen Drucksensor (8, 21) am Pumpeneinlass (41) und/oder am Pumpenauslass (42).

15. Verfahren zum Betrieb einer Herzpumpe nach einem der vorhergehenden Ansprüche, bei dem ein zeitabhängiger Druck des zu fördernden Blutes unmittelbar mittels wenigstens eines am Pumpeneinlass und/oder am Pumpenauslass angeordneten Drucksensors gemessen wird.

**Fig. 1**

**Fig. 2**

**Fig. 3**

**Fig. 4**

Fig. 5

Fig. 6

EKG

AV schließt

AV öffnet

Druck (mm Hg)

100

Aorta

LV

50

MV öffnet

39

MV schließt

40

LA

LVEDP

Zeit

t

| DIASTOLE | SYSTOLE | DIASTOLE |
|---|---|---|
| 36 | 37 | 38 |

Fig. 7

Europäisches
Patentamt

European
Patent Office

Office européen
des brevets

**EUROPÄISCHER RECHERCHENBERICHT**

Nummer der Anmeldung

EP 15 18 2128

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2015/040221 A2 (BERLIN HEART GMBH [DE]) 26. März 2015 (2015-03-26) * Zusammenfassung * * Seite 2, Zeile 36 - Seite 3, Zeile 36 * * Seite 10, Zeile 27 - Zeile 33 * * Seite 20, Zeile 14 - Zeile 21 * * Seite 24, Zeile 1 - Zeile 28 * ----- | 1-8,10, 12,13 | INV. A61M1/10 |
| X | WO 03/015609 A2 (APEX MEDICAL INC [US]) 27. Februar 2003 (2003-02-27) * Zusammenfassung; Abbildungen * * Seite 3, Zeile 6 - Zeile 21 * * Seite 21, Zeile 18 - Zeile 20 * * Seite 5, Zeile 19 - Zeile 23 * * Seite 7, Zeile 30 - Zeile 35 * * Seite 10, Zeile 33 - Seite 11, Zeile 5 * * Seite 12, Zeile 17 - Zeile 23 * ----- | 1,9,11, 14,15 | |
| X | US 2015/025328 A1 (KHAIR MOHAMMAD [US]) 22. Januar 2015 (2015-01-22) * Zusammenfassung; Abbildungen * ----- | 1 | RECHERCHIERTE SACHGEBIETE (IPC) |
| A | US 2006/211909 A1 (ANSTADT MARK P [US] ET AL) 21. September 2006 (2006-09-21) * Absatz [0652] * ----- | 1-15 | A61M |

Der vorliegende Recherchenbericht wurde für alle Patentansprüche erstellt

| Recherchenort | Abschlußdatum der Recherche | Prüfer |
|---|---|---|
| Den Haag | 25. Februar 2016 | Walvoort, Bert |

**ANHANG ZUM EUROPÄISCHEN RECHERCHENBERICHT
ÜBER DIE EUROPÄISCHE PATENTANMELDUNG NR.**

EP 15 18 2128

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-02-2016

| Im Recherchenbericht angeführtes Patentdokument | Datum der Veröffentlichung | Mitglied(er) der Patentfamilie | Datum der Veröffentlichung |
|---|---|---|---|
| WO 2015040221 A2 | 26-03-2015 | EP 2851099 A1<br>WO 2015040221 A2 | 25-03-2015<br>26-03-2015 |
| WO 03015609 A2 | 27-02-2003 | AU 2002331563 A1<br>US 2003045772 A1<br>WO 03015609 A2 | 03-03-2003<br>06-03-2003<br>27-02-2003 |
| US 2015025328 A1 | 22-01-2015 | US 2015018632 A1<br>US 2015025328 A1<br>US 2015031969 A1 | 15-01-2015<br>22-01-2015<br>29-01-2015 |
| US 2006211909 A1 | 21-09-2006 | CA 2530574 A1<br>CN 1838971 A<br>EP 1644060 A2<br>JP 2007524464 A<br>US 2004267086 A1<br>US 2005234289 A1<br>US 2006142634 A1<br>US 2006211909 A1<br>WO 2005000160 A2 | 06-01-2005<br>27-09-2006<br>12-04-2006<br>30-08-2007<br>30-12-2004<br>20-10-2005<br>29-06-2006<br>21-09-2006<br>06-01-2005 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **SARAZAN ; KROEHLE ; MAIN.** Left ventricular pressure, contractility and dP/dt(max) in nonclinical drug safety assessment studies. *Journal of Pharmacological and Toxicological Methods,* 2012
- **VALSECCHI et al.** Cardiac output derived from left ventricular pressure during conductance catheter evaluations: an extended model flow method. *Journal of Clinical Monitoring and Computing,* 2007

- **KARAMANOGLU.** Estimation of cardiac output in patients with congestive heart failure by analysis of right ventricular pressure waveforms. *Biomedical Engineering Online,* 2011